# EUROPÄISCHE PATENTSCHRIFT

(11) **EP 0 845 283 B1**
(45) Veröffentlichungstag und Bekanntmachung des Hinweises auf die Patenterteilung: **15.10.2003**
(21) Anmeldenummer: 97250310.6
(22) Anmeldetag: 21.10.1997
(51) Int. Cl.: A61N 1/375

(54) **Implantat**
implant
implant

(30) Priorität: 23.10.1996 DE 19645371
(43) Veröffentlichungstag der Anmeldung: 03.06.1998
(73) Patentinhaber: BIOTRONIK Mess- und Therapiegeräte GmbH & Co Ingenieurbüro Berlin, 12359 Berlin (DE)
(72) Erfinder: Benz, Hans-Thomas, 91054 Buckenhof (DE); Fischer, Gerhard, Dr., 85716 Unterschleissheim (DE)
(74) Vertreter: Christiansen, Henning, Dipl.-Ing.

(56) Entgegenhaltungen:
- US-A- 4 006 748
- US-A- 4 041 955
- US-A- 4 991 582
- US-A- 5 264 843
- US-A- 5 658 321

## Beschreibung

Die Erfindung betrifft ein in menschliches Gewebe einsetzbares Implantat der im Oberbegriff des ersten Anspruchs genannten Art. Ein derartiges Implantat geht aus der DE 27 20 011 C2 hervor.

Bei in menschliches Gewebe eingesetzten Implantaten, beispielsweise bei einem Herzschrittmacher, muß ein in Kontakttreten der elektronischen Bauteile mit der salzhaltigen und dadurch leitfähigen Körperflüssigkeit vermieden werden, da ansonsten Korrosionserscheinungen und elektrische Fehlfunktionen des Herzschrittmachers auftreten. Dazu ist ein hermetisch verschlossenes Metallgehäuse vorgesehen, welches die empfindlichen elektronischen Baugruppen des Herzschrittmachers in ausreichendem Maße schützt. Eine derartige metallische Kapselung führt jedoch in nachteiliger Weise beim Betrieb des Herzschrittmachers zu einer mehr oder weniger starken Abschirmung magnetischer und elektromagnetischer Wechselfelder, über welche ein Energie- bzw. Signaleintrag oder -austrag in den oder aus dem Herzschrittmacher vorgenommen wird. Dieser im wesentlichen durch Wirbelströme bedingte Energieverlust macht sich einerseits durch eine, auch von dem Patienten häufig als physiologisch störend empfundene, Erwärmung des Gehäuses bemerkbar und erfordert für eine sichere Signalübertragung über eine bestimmte Entfernung andererseits eine größere Energiemenge. Dies stellt insbesondere für den im Herzschrittmacher vorhandenen Energiespeicher eine starke Belastung dar, welche in in nachteiliger Weise zu einer Verkürzung der Entladungszeit des verwendeten Energiespeichers führt. Die Wirbelstromverluste nehmen mit steigender Frequenz zu und begrenzen neben der übertragbaren Datenrate auch die Reichweite der telemetrischen Datenübertragung.

Die Größe der Wirbelströme wird bestimmt durch die Leitfähigkeit und die Dicke des Gehäusematerials. Je dicker das Material und größer seine Leitfähigkeit, um so größer sind die durch telemetrische Übertragungen ausgelöste Wirbelströme.

Aus der eingangs genannten DE 27 20 011 C2 ist ein Herzschrittmacher der wiederaufladbaren Art bekannt, dessen glatte, polierte Gehäusewandung aus einem biokompatiblen Material besteht und eine gleichmäßige Dicke im Bereich von 0,05 mm bis 0,13 mm aufweist. Der Energieeintrag in den Herzschrittmacher zum Aufladen der in dem Gehäuse befindlichen Batterie erfolgt über ein äußeres magnetisches Wechselfeld, wobei die Energieverluste, welche in störender Weise zu einer durch Wirbelströme hervorgerufenen Gehäuseerwärmung führen, durch eine spezielle maßliche Dimensionierung herabgesetzt werden sollen. Daher wird ein Quotient aus Materialstärke und spezifischem elektrischen Widerstand des für das Schrittmachergehäuse verwendeten metallischen Werkstoffs unterhalb eines vorgebenen Grenzwertes gewählt.

Da dieser Quotient bei einem gewählten, biokompatiblen metallischen Werkstoff nur noch durch die Materialstärke der Gehäusewandung bestimmt ist, sind der Anwendung dieser Lösung in nachteiliger Weise Grenzen gesetzt. Diese Grenzen sind grundsätzlich durch eine für den sicheren Betrieb des Herzschrittmachers, d.h. mechanische Festigkeit und Dichtheit gegenüber der den Herzschrittmacher umgebenden Körperflüssigkeit, erforderlichen Mindest-Wandstärke des Gehäuses vorgegeben.

Ausgehend von den Mängeln des Standes der Technik liegt der Erfindung deshalb die Aufgabe zugrunde, einen Implantat der eingangs genannten Gattung anzugeben, dessen Gehäusekonstruktion bei einer für einen sicheren Betrieb des Implantats ausreichenden mechanischen Festigkeit eine weitere Senkung der Energieverluste bei der Signalübertragung in oder aus dem Implantat ermöglicht.

Die Aufgabe wird durch die kennzeichnenden Merkmale des Anspruchs 1 gelöst.

Die Erfindung schließt die Erkenntnis ein, daß die Dämpfungswirkung eines metallischen, hermetisch abgeschlossenen Gehäuses für elektromagnetische Wechselfelder bei einer zur Programmierung erforderlichen telemetrischen Signalübertragung in ein bzw. aus einem Implantat in vorteilhafter und zugleich einfacher Weise herabgesetzt werden kann, wenn die Gehäusewandung mit einer Strukturierung versehen wird, weiche insbesondere einer ungestörten Ausbreitung von in der Gehäusewandung bei der Signalübertragung induzierten Wirbelströmen entgegenwirkt. Diese Strukturierung betrifft einerseits die mechanische Ausbildung der Wandung zur Dickenreduzierung und andererseits eine Veränderung des metallischen Gefüges des Wandungsmaterials zur Verringerung der elektrischen Leitfähigkeit.

Entsprechend der Erfindung weist das Gehäuse des, vorzugweise als Herzschrittmacher ausgebildeten, Implantats eine Wandung auf, in welcher nutenförmig ausgebildete Ausnehmungen vorhanden sind. Diese Ausnehmungen bilden Gehäusebereiche mit einer verringerten metallischen Wandungsstärke, welche von Bereichen mit einer größeren Wandungsstärke eingeschlossen sind, und weisen - bedingt durch ihre Geometrie - einen hohen elektrischen, die Größe induzierter Wirbelströme herabsetzenden Widerstand auf. Diese Ausnehmungen befinden sich an der Innenseite der Gehäusewandung.

Um die durch die Nutung herabgesetzte mechanische Stabilität der Gehäusewandung im wesentlichen wieder auf das normale, für eine gesicherte Betriebsführung des Herzschrittmachers erforderliche Größe zu erhöhen, sind die nutenförmigen Ausnehmungen in der Gehäusewandung mit einem elektrisch nichtleitenden, mechanisch festem Werkstoff ausgefüllt.

Die in den nutenförmigen Ausnehmungen vorhandene Füllung besteht nach einer günstigen Weiterbildung aus einem Kunststoff, vorzugsweise aus einem Epoxidharz, welcher biokompatible und resistent gegenüber der den implantierten Herzschrittmacher umgebenden Körperflüssigkeit ist.

Die Verwendung eines Epoxidharzes sichert durch Klebwirkung in vorteilhafter Weise eine feste, für die mechanische Stabilität der Wandung des Schrittmachergehäuses notwendige Verbindung zwischen der Füllmasse und den Seitenwänden der nutenförmigen Ausnehmungen.

Entsprechend einer bevorzugten Ausführungsform der Erfindung sind die Nuten quaderförmig ausgebildet und erstrecken sich - bei gleichmäßiger Verteilung - im wesentlichen parallel zueinander quer zur Längsachse des Kopfstücks des Herzschrittmachers über die gesamte Länge der jeweiligen Breitseite des Gehäuses. Die Kantenbereiche der quaderförmigen Nuten sind verrundet ausgebildet, um lokale Extremwerte mechanischer Spannungen zu vermeiden und das vollständige Füllen der Ausnehmungen mit einem Kunststoff zu erleichtern.

Nach einer günstigen Weiterbildung der Erfindung bestehen die Ausnehmungen in der Gehäusewandung aus quaderförmigen Nuten mit halbzylindrisch ausgebildeten Enden, wobei mehrere, vorzugsweise zwei, auf gleicher Achse liegende Nutabschnitte mit unterschiedlicher Länge eine sich quer zur Längsachse des Kopfstückes erstreckende Reihe bilden. Auf den Breitseiten des Schrittmachergehäuses sind jeweils eine Mehrzahl der aus nutenförmigen Ausnehmungen gebildeten Reihen parallel zueinander angeordnet. Um die mechanische Stabilität der durch die Ausnehmungen geschwächten Gehäusewandung zu verbessern, ist es günstig, die Position des beispielsweise längeren Nutabschnitts von Reihe zu Reihe zu wechseln.

Entsprechend einer anderen vorteilhaften Ausführungsform der Erfindung sind die nutenförmigen Ausnehmungen nur in dem Wandungsbereich des Schrittmachergehäuses vorhanden, hinter dem die für die Signalübertragung in den bzw. aus dem Herzschrittmacher erforderlichen elektronischen Baugruppen innerhalb des Gehäuses positioniert sind. Diese technologisch günstige Ausführungsform ist möglich, da die exakte Position des Herzschrittmachers vor der Signalübertragung feststellbar ist und die Programmierköpfe der zum Programmieren eines Herzschrittmachers verwendeten elektronischen Geräteeinheiten nur eine relativ geringe Größe aufweisen.

Entsprechend zusätzlichen Weiterbildungen der Erfindung sind die nutenförmigen Ausnehmungen sowohl an der Innenseite als auch an der Außenseite oder nur an der Außenseite der Gehäusewandung vorhanden.

Nach der bevorzugten Ausführungsform ist die Materialstärke der Wandung des Schrittmachergehäuses in den nutenförmigen Ausnehmungen um mindestens 60%, vorzugsweise um 80%, gegenüber den nicht genuteten Wandungsbereichen reduziert.

Entsprechend einer anderen vorteilhaften Ausführungsform der Erfindung weist das für die Gehäusewandung eingesetzte, als Metallblech ausgebildete Material örtliche Veränderungen der Materialstruktur auf, welche mittels lokaler Oxidation durch Sauerstoffimplantation, durch lokale Legierungsbildung oder durch lokale Implantation von Störstellen erzeugt worden sind und zu einer Reduzierung der elektrischen Leitfähigkeit des Implantatgehäuses führen. Dadurch ist eine zusätzliche Reduzierung der Wirbelströme erreichbar.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Seitenansicht einer bevorzugten Ausführungsform der Erfindung,
- Figur 2: die Darstellung der Ansicht eines Schnittes längs der Linie A...A gemäß Figur 1,
- Figur 3: die Darstellung der Ansicht eines Schnittes längs der Linie B...B gemäß Figur 1,
- Figur 4: eine Seitenansicht einer anderen vorteilhaften Ausführungsform der Erfindung,
- Figur 5: die Ansicht des Schnittes längs der Linie C...C gemäß Figur 4,
sowie
- Figur 6: eine günstige Weiterbildung der Erfindung in Ansicht von der Seite.

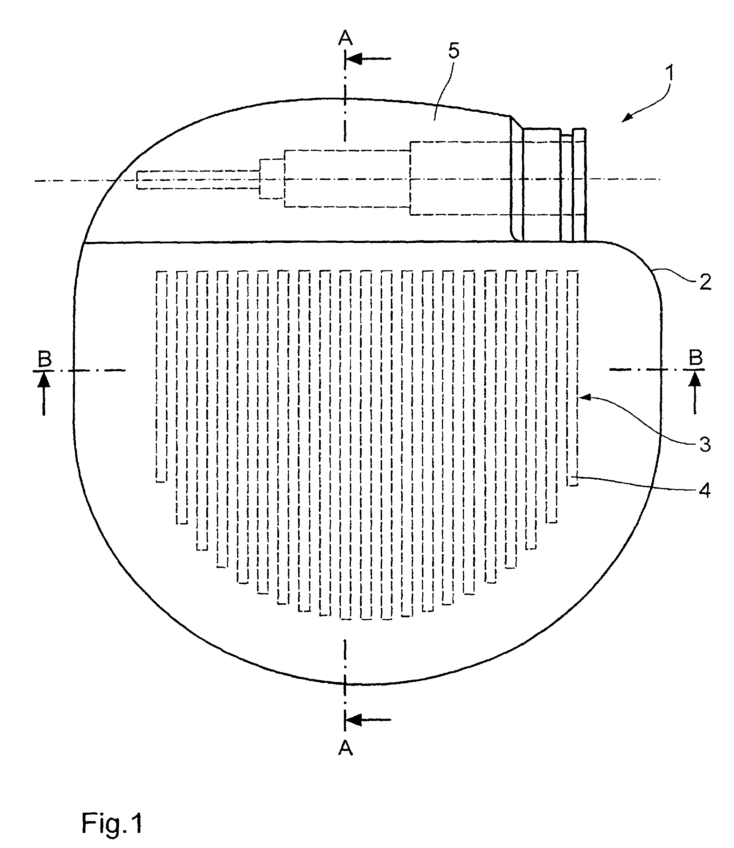

In Figur 1 ist ein Herzschrittmacher 1 in Seitenansicht schematisiert dargestellt. Das napfförmige Gehäuse 2 ist durch ein Kopfstück 5 verschlossen, in welchem eine Steckbuchse 10 zum Anschluß einer (nicht dargestellten) Stimulationsleitung angeordnet ist. An den Breitseiten des Gehäuses 2 sind nutenförmige Ausnehmungen 3 vorhanden, welche sich senkrecht zur Längsachse 9 des Kopfstücks 5 erstrekken. Die an der Innenseite der Gehäusewandungen befindlichen, nutenförmigen Ausnehmungen 3 sind quaderförmig ausgebildet, erstrecken sich zueinander parallelliegend und sind gleichmäßig über die gesamte Breitseite des Gehäuses 2 verteilt angeordnet. Die nutenförmigen Ausnehmungen sind bei der relativ geringen Materialstärke der Gehäusewandung durch eine spanlose Formgebung der Gehäusehälften oder durch eine Laserbearbeitung herstellbar.

In die nutenförmigen Ausnehmungen 3 ist eine elektrisch nichtleitende Füllung 4 eingebracht, welche die Nuten 3 vollständig ausfüllt und mit den Nutwandungen eine Haftverbindung eingeht. Die Füllung 4 besteht aus einem Epoxidharz, welches mit der Nutwandung verklebt ist. Durch die Epoxidharz-Füllung 4 wird die durch die Nutung 3 der Gehäusewandung 2 bedingte mechanische Instabilität nahezu vollständig ausgeglichen. Das Gehäuse 2 des Herzschrittmachers 1 ist gasdicht, und die Ausbildung von Wirbelströmen in der Gehäusewandung 2 bei der Signalübertragung vom oder zum Herzschrittmacher wird weitgehend unterdrückt.

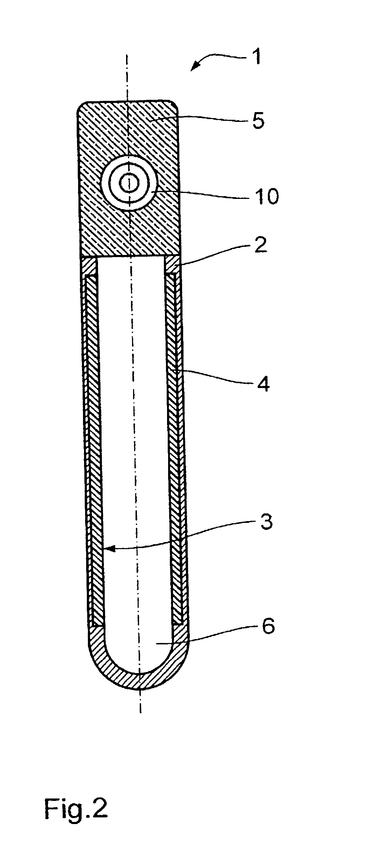

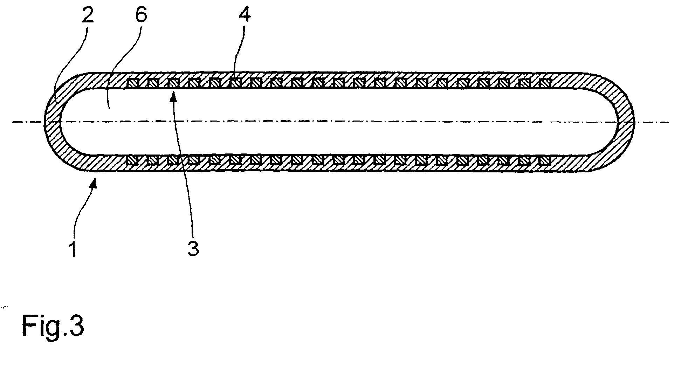

Die Darstellung in den Figuren 2 und 3 zeigt die Form der nutenförmigen Ausnehmungen 3 in der Wandung des Gehäuses 2. Die an der Innenseite der Gehäusewandung vorhandenen Ausnehmungen 3 sind quaderförmig ausgebildet und weisen verrundete Kantenbereiche auf. Dadurch werden lokale Extremwerte mechanischer Spannungen vermieden und das vollständige Füllen der Ausnehmungen mit einem Kunststoff erleichtert.

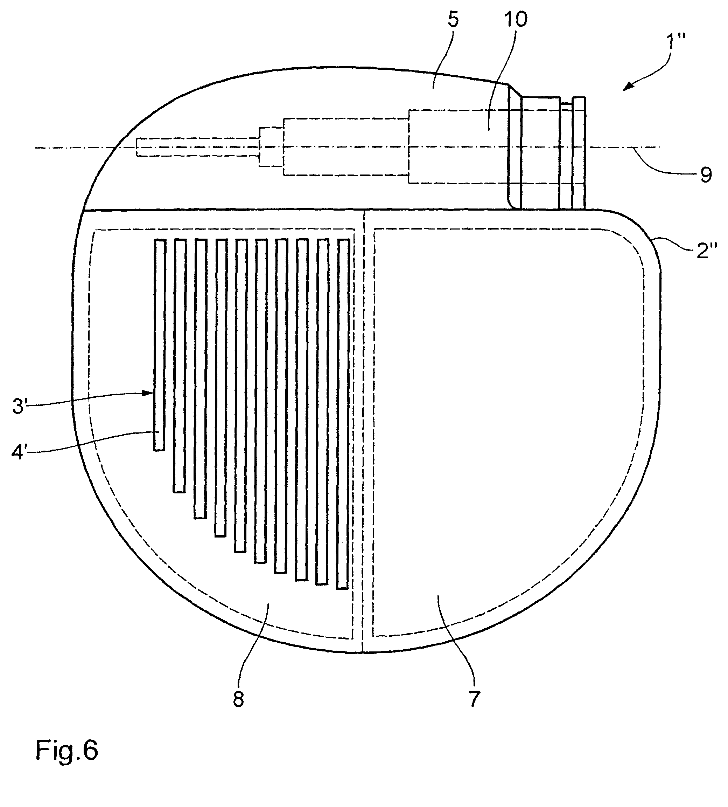

In dem Gehäuseinnenraum 6 sind ein Energiespeicher (vergleiche die Position 7 in Figur 6) und elektronische Mittel zur hochfrequenten Informationsübertragung (vergleiche die Position 8 in Figur 6) von einem Programmiergerät in den Implantat hinein und aus diesem heraus zu dem Programmiergerät angeordnet.

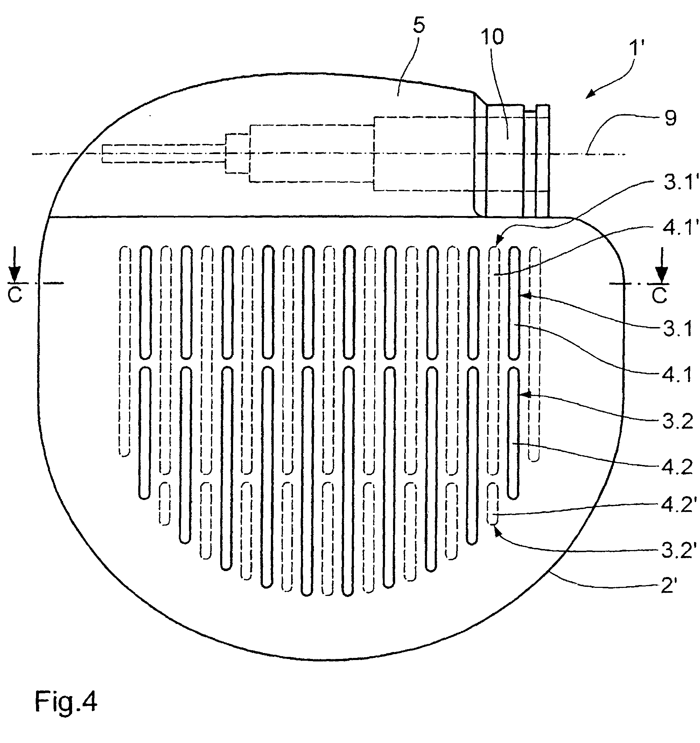

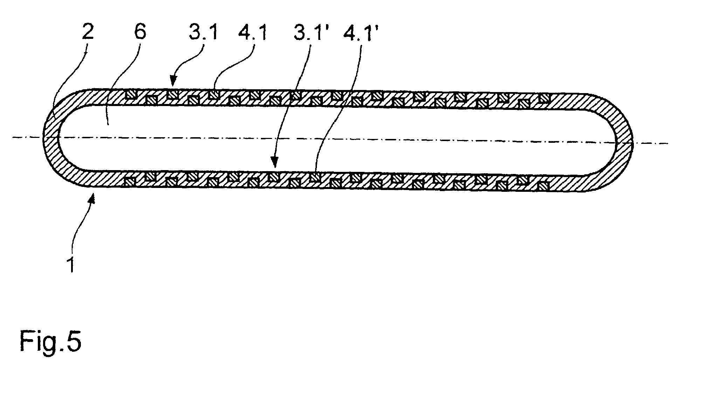

Das in den Figuren 4 und 5 in Seiten- und Schnittansicht schematisiert dargestellte Gehäuse 2' eines Herzschrittmachers 1' weist in der Wandung der Gehäusebreitseiten nutenförmige Ausnehmungen auf, welche abwechselnd auf der Innenund Außenseite der Gehäusewandung angeordnet und in unterschiedlich lange Abschnitte 3.1 und 3.2 bzw. 3.1' und 3.2' untergliedert sind.

Die Abschnitte 3.1 und 3.2 bzw. 3.1' und 3.2' liegen auf gleicher Achse und sind an ihren Enden halbzylindrisch ausgebildet. An allen Kantenbereichen sind Verrundungen vorhanden. Um die mechanische Stabilität der durch die Ausnehmungen 3.1 und 3.2 bzw. 3.1' und 3.2' geschwächten Gehäusewandung zu verbessern, wird die Position der längeren Nutabschnitte 3.2 und 3.2' in vorteilhafter Weise von Reihe zu Reihe gewechselt. Ebenso wie die in Figur 1 gezeigte Ausführungsform der Erfindung weisen die nutenförmigen Ausnehmungen 3.1 und 3.2 bzw. 3.1' und 3.2' jeweils eine Füllung 4.1, 4.2, 4.1', 4.2' mit einem Epoxidharz auf, so daß die Außenwandung des Schrittmachergehäuses eine gleichförmige, glatte Oberfläche und im wesentlichen die gleiche mechanische Stabilität aufweist wie eine ungenutete Wandung.

Die in Figur 6 dargestellte Ausführungsform der Erfindung 1" weist ein Gehäuse 2" auf, an dessen Wandung außenseitig eine Nutung 3 lediglich in den Bereichen des Gehäuses 2" vorhanden ist, hinter denen sich die elektronischen Mittel 8 zur hochfrequenten telemetrischen Informationsübertragung von einem Programmiergerät in den Herzschrittmacher 1" hinein und aus diesem heraus zu dem Programmiergerät befinden. Die Gestaltung der nutenförmigen Ausnehmungen 3' und ihre Füllung 4' entspricht der der vorstehend beschriebenen Ausführungsformen.

## Patentansprüche

1. In menschliches Gewebe einsetzbares Implantat (1, 1', 1''), insbesondere Herzschrittmacher, mit einem hermetisch dichten, metallischen Gehäuse (2, 2', 2*''*), mit in diesem Gehäuse eingeschlossenen elektronischen Mitteln (8) zur hochfrequenten, telemetrischen Informationsübertragung zwischen einem Programmiergerät und dem Implantat sowie mit einem Kopfstück (5) zum Anschluß einer Stimulationsleitung,
**dadurch gekennzeichnet,**
**daß** die Wandung des Gehäuses (2, 2', 2") die metallische Wandstärke reduzierende, nutenförmige Ausnehmungen (3, 3', 3.1, 3.1', 3.2, 3.2') derart aufweist, daß in der Gehäusewandung im Bereich der Ausnehmungen (3, 3', 3.1, 3.1', 3.2, 3.2') eine die unter Einsatzbedingungen erforderliche Flüssigkeitsdichtheit des Gehäuses (2, 2', 2") sichernde Materialstärke nicht unterschritten wird.

2. Implantat nach Anspruch 1, **dadurch gekennzeichnet, daß** die nutenförmigen Ausnehmungen (3, 3.1, 3.1', 3.2, 3.2') an einer Wandungsinnen- und/oder Wandungsaußenseite des Gehäuses (2, 2') angeordnet sind.

3. Implantat nach Anspruch 2, **dadurch gekennzeichnet, daß** die nutenförmigen Ausnehmungen (3, 3.1', 3.2') an einer Wandungsinnenseite des Gehäuses (2, 2') angeordnet sind.

4. Implantat nach einem der vorhergehenden Ansprüche, **dadurch gekennzeichnet, daß** die nutenförmigen Ausnehmungen (3') an einem die elektronischen Mittel (8) der Telemetriestrecke bedeckenden Wandungsbereich des Gehäuses (2") angeordnet sind.

5. Implantat nach Anspruch 1, **dadurch gekennzeichnet, daß** die nutenförmigen Ausnehmungen (3, 3', 3.1, 3.1', 3.2, 3.2') eine Füllung (4, 4', 4.1, 4.1', 4.2, 4.2') mit einem elektrisch nichtleitenden Werkstoff aufweisen.

6. Implantat nach Anspruch 5, **dadurch gekennzeichnet, daß** die Füllung (4, 4', 4.1, 4.1', 4.2, 4.2') aus Kunststoff besteht.

7. Implantat nach Anspruch 6, **dadurch gekennzeichnet, daß** die Kunststoffüllung (4, 4', 4.1, 4.1', 4.2, 4.2') aus Polyesterharz besteht.

8. Implantat nach Anspruch 1, **dadurch gekennzeichnet, daß** sich die nutenförmigen Ausnehmungen (3, 3', 3.1, 3.1', 3.2, 3.2') im wesentlichen parallel zueinander und quer zur Längsachse (9) des Kopfstücks (5) erstrecken.

9. Implantat nach Anspruch 8, **dadurch gekennzeichnet, daß** die nutenförmigen Ausnehmungen (3, 3') jeweils in zwei auf gleicher Achse liegende Abschnitte (3.1, 3.2 bzw. 3.1', 3.2') unterschiedlicher Länge unterteilt sind.

10. Implantat nach Anspruch 9, **dadurch gekennzeichnet, daß** in jeder Ausnehmungsreihe die Reihenfolge der ungleich langen Abschnitte (3.1, 3.2 bzw. 3.1', 3.2') wechselt.

11. Implantat nach Anspruch 1, **dadurch gekennzeichnet, daß** die Stärke der metallischen Wandung im Bereich der Ausnehmungen (3, 3', 3.1, 3.1', 3.2, 3.2') um mindestens 60%, vorzugsweise um 80%, reduziert ist.

12. Implantat nach einem der vorhergehenden Ansprüche, **dadurch gekennzeichnet, daß** sich die nutenförmigen Ausnehmungen (3, 3', 3.1, 3.1', 3.2, 3.2') über die gesamte Breitseite des Schrittmachergehäuses (2, 2', 2") erstrekken und gleichmäßig verteilt angeordnet sind.

13. Implantat nach einem der vorhergehenden Ansprüche, **dadurch gekennzeichnet, daß** die nutenförmigen Ausnehmungen (3, 3', 3.1, 3.1', 3.2, 3.2') im wesentlichen quaderförmig ausgebildet sind.

14. Implantat nach Anspruch 13, **dadurch gekennzeichnet, daß** die nutenförmigen Ausnehmungen (3.1, 3.1', 3.2, 3.2') halbzylindrisch geformte Endbereiche aufweisen.

15. Implantat nach einem der vorhergehenden Ansprüche, **dadurch gekennzeichnet, daß** das für das Gehäuse (2, 2', 2") verwendete Material Bereiche verminderter elektrischer Leitfähigkeit aufweist, welche durch lokale Sauerstoffimplantation, lokale Legierungsbildung oder durch Einbringen lokaler Störstellen in das Gefüge erzeugt worden sind.

16. Implantat nach einem der vorhergehenden Ansprüche, **dadurch gekennzeichnet, daß** das Gehäuse (2, 2', 2") aus Titan besteht.

## Claims

1. An implant (1, 1', 1") which can be inserted into human tissue, in particular a cardiac pacemaker, comprising a hermetically sealed metal housing (2, 2', 2"), electronic means (8) enclosed in said housing for highfrequency telemetric information transmission between a programming device and the implant, and a head portion (5) for the connection of a stimulation line, **characterised in that** the wall of the housing (2, 2, 2") has groove-shaped recesses (3, 3', 3.1, 3.1', 3.2, 3.2') reducing the metal wall thickness, in such a way that in the housing wall in the region of the recesses (3, 3', 3.1, 3.1', 3.2, 3.2') the material thickness is not less than a thickness ensuring the fluid sealing integrity of the housing (2, 2', 2") which is required under conditions of use.

2. An implant according to claim 1 **characterised in that** the groove-shaped recesses (3, 3.1, 3.1', 3.2, 3.2') are arranged at an inside and/or outside of the wall of the housing (2, 2').

3. An implant according to claim 2 **characterised in that** the groove-shaped recesses (3, 3.1', 3.2') are arranged at an inside of the wall of the housing (2, 2').

4. An implant according to one of the preceding claims **characterised in that** the groove-shaped recesses (3') are arranged at a wall region of the housing (2"), which covers the electronic means (8) of the telemetry arrangement.

5. An implant according to claim 1 **characterised in that** the groove-shaped recesses (3, 3', 3.1, 3.1', 3.2, 3.2') have a filling (4, 4', 4.1, 4.1', 4.2, 4.2') with an electrically non-conductive material.

6. An implant according to claim 5 **characterised in that** the filling (4, 4', 4.1, 4.1', 4.2, 4.2') comprises plastic material.

7. An implant according to claim 6 **characterised in that** the plastic filling (4, 4', 4.1, 4.1', 4.2, 4.2') comprises polyester resin.

8. An implant according to claim 1 **characterised in that** the groove-shaped recesses (3, 3', 3.1, 3.1', 3.2, 3.2') extend substantially parallel to each other and transversely with respect to the longitudinal axis (9) of the head portion (5).

9. An implant according to claim 8 **characterised in that** the groove-shaped recesses (3, 3') are each subdivided into two portions (3.1, 3.2 and 3.1', 3.2' respectively) of differing lengths, which are on the same axis.

10. An implant according to claim 9 **characterised in that** the sequence of the portions (3.1, 3.2 and 3.1', 3.2') of unequal lengths changes in each row of recesses.

11. An implant according to claim 1 **characterised in that** the thickness of the metal wall in the region of the recesses (3, 3', 3.1, 3.1', 3.2, 3.2') is reduced by at least 60% and preferably by 80%.

12. An implant according to one of the preceding claims **characterised in that** the groove-shaped recesses (3, 3', 3.1, 3.1', 3.2, 3.2') extend over the entire wide side of the pacemaker housing (2, 2', 2") and are arranged uniformly distributed.

13. An implant according to one of the preceding claims **characterised in that** the groove-shaped recesses (3, 3', 3.1, 3.1', 3.2, 3.2') are of a substantially cuboidal shape.

14. An implant according to claim 13 **characterised in that** the groove-shaped recesses (3.1, 3.1', 3.2, 3.2') have end regions of a semicylindrical shape.

15. An implant according to one of the preceding claims **characterised in that** the material used for the housing (2, 2', 2") has regions of reduced electrical conductivity which have been produced by local oxygen implantation, local alloy formation or by the introduction of local faults into the structure.

16. An implant according to one of the preceding claims **characterised in that** the housing (2, 2, 2") comprises titanium.

## Revendications

1. Implant (1, 1', 1") pouvant être inséré dans un tissu humain, notamment stimulateur cardiaque, comportant un boîtier métallique (2, 2', 2") hermétiquement étanche, comportant des moyens électroniques (8) renfermés dans ce boîtier pour réaliser la transmission télémétrique d'informations, à haute fréquence, entre un appareil de programmation et l'implant, et comportant une partie de tête (5) pour le raccordement d'un conducteur de stimulation,
**caractérisé en ce**
**que** la paroi du boîtier (2, 2', 2") possède des évidements (3, 3', 3.1, 3.1', 3.2, 3.2') en forme de rainures, qui réduisent l'épaisseur de la paroi métallique de telle sorte que dans la paroi du boîtier, dans la zone des évidements (3, 3', 3.1, 3.1', 3.2, 3.2'), l'épaisseur du matériau n'est pas inférieure à une épaisseur de matériau garantissant l'étanchéité du boîtier (2, 2', 2") aux liquides, qui est nécessaire dans des conditions d'utilisation.

2. Implant selon la revendication 1, **caractérisé en ce que** les évidements en forme de rainures (3, 3.1, 3.1', 3.2, 3.2') sont disposés sur un côté intérieur et/ou un côté extérieur de la paroi du boîtier (2, 2').

3. Implant selon la revendication 2, **caractérisé en ce que** les évidements en forme de rainures (3, 3.1', 3.2') sont disposés sur un côté intérieur de la paroi du boîtier (2, 2').

4. Implant selon l'une des revendications précédentes, **caractérisé en ce que** les évidements en forme de rainures (3') sont disposés dans un élément de paroi du boîtier (2"), qui recouvre les moyens électroniques (8) de la section de télémétrie.

5. Implant selon la revendication 1, **caractérisé en ce que** les évidements en forme de rainures (3, 3', 3.1, 3.1', 3.2, 3.2') comportent un remplissage (4, 4', 4.1, 4.1', 4.2, 4.2') comprenant un matériau électriquement non conducteur.

6. Implant selon la revendication 5, **caractérisé en ce que** le remplissage (4, 4', 4.1, 4.1', 4.2, 4.2') est réalisé en matière plastique.

7. Implant selon la revendication 6, **caractérisé en ce que** le remplissage en matière plastique (4, 4', 4.1, 4.1', 4.2, 4.2') est formé d'une résine polyester.

8. Implant selon la revendication 1, **caractérisé en ce que** les évidements en forme de rainure (3, 3', 3.1, 3.1', 3.2, 3.2') s'étendent essentiellement parallèlement entre eux et transversalement par rapport à l'axe longitudinal (9) de l'élément de tête (5).

9. Implant selon la revendication 8, **caractérisé en ce que** les évidements en forme de rainures (3, 3') sont divisés respectivement en deux sections (3.1, 3.2 ou 3.1', 3.2') situées sur le même axe et possédant des longueurs différentes.

10. Implant selon la revendication 9, **caractérisé en ce que** dans chaque rangée d'évidements, la succession des sections ayant des longueurs différentes (3.1, 3.2, ou 3.1', 3.2') est alternée.

11. Implant selon la revendication 1, **caractérisé en ce que** l'épaisseur de la paroi métallique dans la zone des évidements (3, 3', 3.1, 3.1', 3.2, 3.2') est réduite d'au moins 60 % et de préférence de 80 %.

12. Implant selon l'une des revendications précédentes, **caractérisé en ce que** les évidements en forme de rainures (3, 3', 3.1, 3.1', 3.2, 3.2') s'étendent sur l'ensemble du côté large du boîtier (2, 2', 2") du stimulateur et sont disposés en étant répartis uniformément.

13. Implant selon l'une des revendications précédentes, **caractérisé en ce que** les évidements en forme de rainures (3, 3', 3.1, 3.1', 3.2, 3.2') sont réalisés avec une forme essentiellement parallélépipédique.

14. Implant selon la revendication 13, **caractérisé en ce que** les évidements en forme de rainures (3.1, 3.1', 3.2, 3.2') comportent des parties d'extrémité de forme semi-cylindrique.

15. Implant selon l'une des revendications précédentes, **caractérisé en ce que** le matériau utilisé pour le boîtier (2, 2', 2") comporte des parties ayant une conductivité électrique réduite, qui ont été produites dans la structure par une implantation locale d'oxygène, une formation locale d'alliage ou une insertion de défauts locaux.

16. Implant selon l'une des revendications précédentes, **caractérisé en ce que** le boîtier (2, 2', 2") est réalisé en titane.
